# EUROPEAN PATENT APPLICATION

(11) **EP 4 512 884 A1**
(43) Date of publication of application: **26.02.2025**
(21) Application number: 22944051.6
(22) Date of filing: 30.05.2022
(51) Int. Cl.: C12M 1/26

(54) **CELL TRANSFER APPARATUS**

(71) Applicant: Yamaha Hatsudoki Kabushiki Kaisha, Iwata-shi, Shizuoka 438-8501 (JP)
(72) Inventor: WATANABE, Masahiko, Iwata-shi, Shizuoka 438-8501 (JP)
(74) Representative: Müller-Boré & Partner Patentanwälte PartG mbB
(86) International application number: PCT/JP2022/021920
(87) International publication number: WO 2023/233464

(57) **Abstract**

A cell transfer apparatus transfers cells scattered in a first container to a second container. The cell transfer apparatus includes a head to which a suction tip that can generate suction force and discharge force in a distal end opening is attached, a movement mechanism for the head, a camera that captures images of the first container, a cell recognition unit that performs position recognition of the cells based on the images, and a control unit that controls the head and the movement mechanism to perform a transfer operation of the cells based on a result of the position recognition. The control unit gradually suctions the set quantity of target cells that is set to two or more in advance into the suction tip from among the cells scattered in the first container, causes the head to move to the second container, and then discharges the set quantity of the target cells that are suctioned into the suction tip to the same location in the second container.

## Description

### Technical Field

The present invention relates to a cell transfer apparatus for transferring cells scattered in one container to another container.

### Background Art

For example, in the fields of medical and biological research, there are cases where work is performed to transfer cells from a sorting container for sorting cultured single cells or cell colonies (which may be simply referred to as "cells" in this specification) to a work container for inspection, observation, or the like. For this work, a cell transfer apparatus is known that uses a head to which a suction tip is attached to perform the operation of suctioning one target cell from the sorting container and discharging the suctioned target cell into a well of the work container.

Depending on the mode of research, it may be necessary to hold a plurality of cells in one well of the work container. In this case, it is necessary to repeat the operation of causing a suction tip in the source sorting container to suction one cell and discharge the suctioned cell in the destination work container multiple times. That is, it is necessary to perform multiple reciprocating movements of the head between the source container and the destination container, resulting in a longer tact time.

Patent Literature 1 discloses a target object observation device equipped with a suction tip that can suction and hold multiple cells. However, Patent Literature 1 assumes the use of a suction tip with a special shape having a cell trap part with a horizontal part.

### Citation List

### Patent Literature

Patent Literature 1: Japanese Patent No. 5897733

### Summary of Invention

An object of the present invention is to provide a cell transfer apparatus that can efficiently transfer multiple cells scattered in one container to another container.

### Solution to Problem

A cell transfer apparatus according to one aspect of the present invention is a cell transfer apparatus for transferring cells scattered in a first container to a second container, and includes: a head to which a suction tip configured to suction and discharge the cells is attached, the head including a generating mechanism that generates suction force and discharge force in a distal end opening of the suction tip; a movement mechanism that causes the head to perform horizontal movement and up-and-down movement; a cell recognition unit that includes a camera that captures an image of the first container in which the cells are scattered, the cell recognition unit performing position recognition on the cells based on the image; and a control unit that controls the head and the movement mechanism to perform a transfer operation of the cells based on a result of the position recognition, in which the control unit causes the suction tip to gradually suction a set quantity of target cells from among the cells scattered in the first container, the set quantity being set in advance to two or more, and after causing the head to move to the second container, the control unit causes the suction tip to discharge the set quantity of the target cells that is suctioned by the suction tip into a same location in the second container.

### Brief Description of Drawings

FIG. 1 is a schematic diagram roughly showing the configuration of a cell transfer apparatus according to an embodiment of the present invention.
FIG. 2 is a cross-sectional view of a suction tip attached to a head of the cell transfer apparatus.
FIG. 3A is a cross-sectional view of a syringe, which is a component of the suction tip, FIG. 3B is a cross-sectional view of a plunger, and FIG. 3C is an exploded perspective view of the suction tip.
FIG. 4 is a block diagram showing the control configuration of the cell transfer apparatus.
FIGS. 5A to 5D are diagrams showing steps of suctioning cells into the suction tip.
FIGS. 6A to 6D are diagrams showing steps of suctioning cells into the suction tip.
FIGS. 7A to 7C are diagrams showing the transfer step and discharge step of the suctioned cells.
FIGS. 8A and 8B are examples of images before and after cell suction in a first container having accommodation sections, and FIG. 8C is an example of an image of a well in a second container where the cells are discharged.
FIGS. 9A and 9B are examples of images before and after cell suction in the first container having no accommodation sections, and FIG. 9C is an example of an image of the well in the second container where the cells are discharged.
FIG. 10 is a flowchart showing an operation example of the cell transfer apparatus.
FIG. 11 is a flowchart showing the operation of cell transfer steps by the cell transfer apparatus.
FIG. 12 is a flowchart showing details of a cell suction operation by the head.
FIG. 13 is a diagram showing a pattern for confirming success or failure of cell suction.
FIG. 14 is a perspective view showing one example of a cell transfer line assembled in the cell transfer apparatus.

### Description of Embodiments

An embodiment of the present invention will be described in detail below with reference to the drawings. The cell transfer apparatus according to the present invention can suction and transfer various types of cells of biological origin. Examples of the cell of biological origin include single cells such as hematopoietic cells and dissociated cells, tissue fragments such as histoculture, cell aggregation clusters such as spheroids and organoids, individuals such as zebrafish, nematodes, and fertilized eggs, 2D or 3D cell colony, and the like.

### [Overall Configuration of Cell Transfer Apparatus]

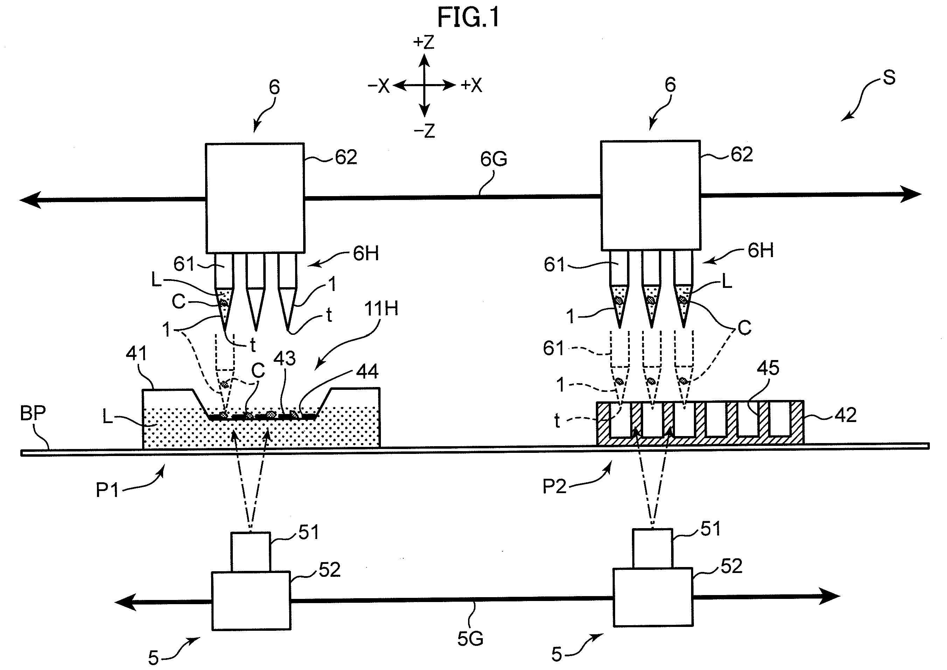

FIG. 1 is a diagram schematically showing the overall configuration of the cell transfer apparatus S. Here, the cell transfer apparatus S that transfers cells C between two containers is illustrated. The cell transfer apparatus S includes a translucent base BP having a horizontal placement surface, a camera unit 5 placed below the base BP, and a head unit 6 placed above the base BP. A first container 41, which serves as the source of transfer of the cells C, is placed at a first placement position P1 of the base BP. A second container 42, which serves as the destination of transfer of the cells C, is placed at a second placement position P2 of the base BP.

The first container 41 includes a dish 43 that holds (arranges) the cells C in a scattered manner. The second container 42 includes numerous wells 45 that can accommodate the cells C. The camera unit 5 and the head unit 6 are movable at least in the X direction. The head unit 6 includes a plurality of heads 61 to each of which a suction tip 1 that suctions and discharges the cells C is attached. The heads 61 are movable in the Z direction.

Generally speaking, the cell transfer apparatus S transfers the cells C from the first container 41 at the first placement position P1 to the second container 42 at the second placement position P2. First, the cell transfer apparatus S sorts target cells C to be transferred from among numerous cells scattered on the dish 43 of the first container 41, and suctions and holds the target cells C with the suction tip 1. The suction tip 1 that holds the cells C moves to the second container 42 together with the head unit 6, and the cells C are discharged into the well 45 of the second container 42. As will be described in detail later, during the suction in the first container 41, the set quantity of the target cells C that is set to two or more in advance is suctioned by the suction tip 1, and the cells C are discharged into one well 45 (same location) of the second container 42. Each part of the cell transfer apparatus S will be described below.

The base BP is a rectangular flat plate having predetermined rigidity, and part or all of which includes a translucent material. The preferred base BP is a glass plate. The base BP includes a translucent material such as a glass plate, thereby allowing the camera unit 5 placed below the base BP to capture images of the first and second containers 41 and 42 placed on the upper surface of the base BP through the base BP.

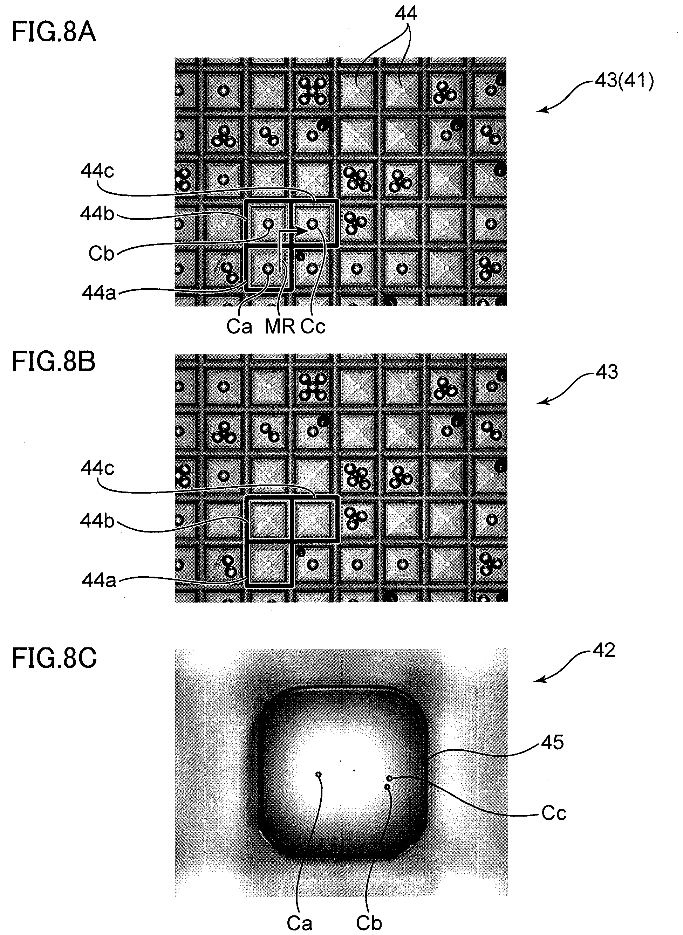

The first container 41 stores a culture medium L, and holds the dish 43 for sorting cells in a state of being immersed in the culture medium L. The dish 43 has multiple accommodation sections 44 that can accommodate the cells C on the upper side. The accommodation sections 44 each include a recess that can accommodate the cells C and are arranged in a matrix. Note that FIGS. 8A and 8B show the upper view of the dish 43. The culture medium L is not particularly limited as long as the culture medium does not deteriorate the properties of the cells C, and can be appropriately selected depending on the type of cells C.

The first container 41 includes a rectangular upper opening 11H on the upper surface side. The upper opening 11H is an opening for injecting the cells C and picking up the sorted cells C. The injection of the cells C is a step of discharging a cell suspension containing a large amount of cells C from a dispenser tip (not shown). The pickup of the cells C is performed by the suction tip 1 described above. The dish 43 is placed below the upper opening 11H. The first container 41 and the dish 43 are produced using a translucent resin material or glass. This is to allow the camera unit 5 placed below the first container 41 to observe the cells C carried in the dish 43.

The second container 42 is, for example, a microplate having multiple wells 45 (accommodation portions). The wells 45 are each a bottomed hole opened on an upper surface of the second container 42. In one well 45, a necessary number of cells C is accommodated together with the culture medium L. The second container 42 is also produced using a translucent resin material or glass for allowing the camera unit 5 to capture images. When discharging the cells C, a distal end t of the suction tip 12 enters the well 45. Each well 45 has an opening diameter that allows the distal end t to enter with room to spare.

The camera unit 5 (part of cell recognition unit) captures images of the cells C held in the first container 41 or the second container 42 from the undersides. The camera unit 5 includes a lens unit 51 and a camera body 52. The lens unit 51 is an object lens used in an optical microscope, and includes a lens group that forms a light image with a predetermined magnification and a lens barrel that accommodates the lens group. The camera body 52 includes an image capturing element such as a CCD image sensor. The lens unit 51 forms a light image of an image capturing target on a light receiving surface of the image capturing element. The camera unit 5 is movable horizontally along a guide rail 5G extending in the X direction. The camera unit 5 may also be movable in the Y direction orthogonal to the paper surface in FIG. 1 by a movement mechanism (not shown).

The head unit 6 is a unit that performs the operation of picking the cells C from the first container 41 for transfer to the second container 42, and includes a head group 6H having a plurality of the heads 61 and a head body 62 to which the head group 6H is assembled. The suction tip 1 that can suction and discharge the cells C is attached to the distal end of each head 61. Each head 61 includes a generating mechanism that generates suction force and discharge force at the distal end t of the suction tip 1. The head body 62 holds the heads 61 to allow up-and-down movement in the +Z and -Z directions. The head unit 6 moves in the X direction where the first container 41 and the second container 42 are arranged along a guide rail 6G (movement mechanism). Actually, the head unit 6 is also movable in the Y direction orthogonal to the paper surface in FIG. 1 by a movement mechanism (not shown). In this way, the head unit 6 functions as a movement mechanism that causes the suction tip 1 to perform horizontal movement in the XY direction and to perform up-and-down movement in the Z direction.

### [Structure of Suction Tip]

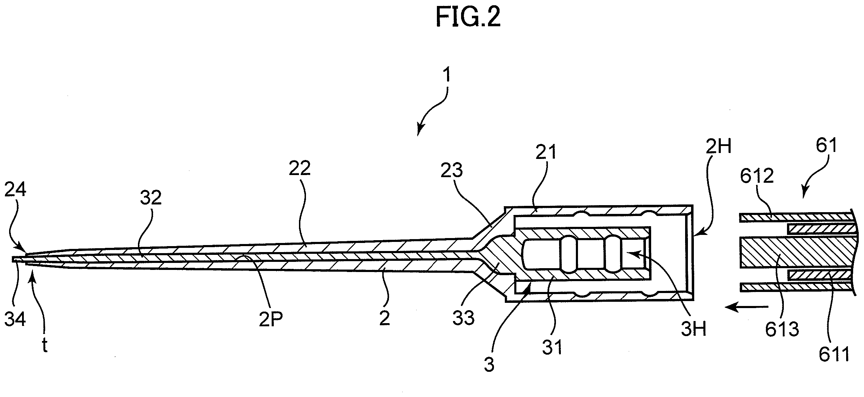

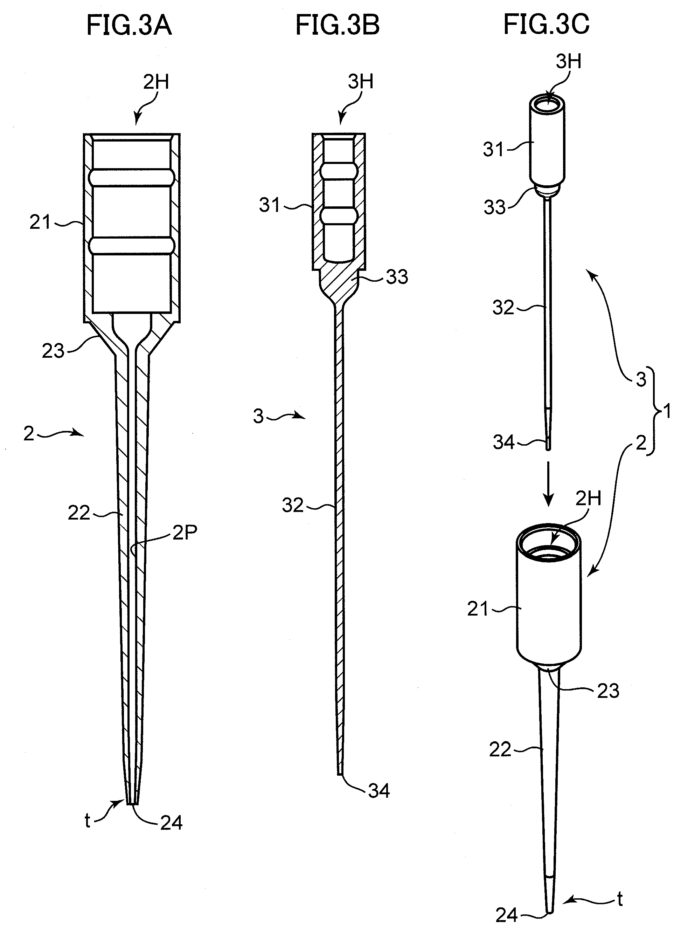

Subsequently, the structure of the suction tip 1 attached to the head 61 will be described. FIG. 2 is a cross-sectional view of the suction tip 1. FIG. 3A is a cross-sectional view of a syringe 2, FIG. 3B is a cross-sectional view of a plunger 3, and FIG. 3C is an exploded perspective view of the suction tip 1. The suction tip 1 includes the syringe 2 with a tubular passage 2P that serves as a suction path for the cells C, and the plunger 3 that is slidably accommodated inside the tubular passage 2P.

The syringe 2 includes a syringe proximal end 21 having a large diameter cylindrical shape, a syringe body 22 having a small diameter long cylindrical shape, and a taper cylinder part 23 that connects the proximal end 21 and the body 22. The tubular passage 2P is formed in the syringe body 22. At the distal end t located at one end of the syringe body 22, a distal end opening 24 that serves as a suction port or discharge port for the cells C is provided. One end of the tubular passage 2P is connected to the distal end opening 24. The syringe proximal end 21 is connected to the other end of the syringe body 22 via the taper cylinder part 23.

The plunger 3 is a member that is inserted into the tubular passage 2P of the syringe 2 and generates negative pressure for cell suction or positive pressure for cell discharge in the distal end opening 24 by moving back and forth inside the tubular passage 2P. The plunger 3 includes a plunger proximal end 31 having a cylindrical shape, a needle-shaped plunger body 32, a dome-shaped part 33 connecting the proximal end 31 and the body 32, and a plunger distal end 34 that is a protruding end of the plunger body 32.

The syringe proximal end 21 includes a cylindrical hollow part 2H. The outer diameter of the plunger proximal end 31 is set to be smaller than the inner diameter of the hollow part 2H by a predetermined length. The outer diameter of the plunger body 32 is set to be slightly smaller than the inner diameter of the tubular passage 2P. The shape of the inner circumferential surface of the taper cylinder part 23 matches the curved surface shape of the outer circumferential surface of the dome-shaped part 33. The plunger 3 is assembled with respect to the syringe 2 in a mode in which the plunger proximal end 31 is accommodated in the hollow part 2H, and the plunger body 32 is inserted into the tubular passage 2P of the syringe body 22.

FIG. 3C shows the state where the plunger 3 is pulled out from the syringe 2, but FIG. 2 shows the state where the plunger body 32 is inserted deepest into the syringe body 22, that is, the state where the plunger 3 has descended the most. In this state, the dome-shaped part 33 is completely accommodated in the cavity of the taper cylinder part 23. The length of the plunger body 32 is slightly longer than the syringe body 22, and in the state shown in FIG. 2, the plunger distal end 34 protrudes from the distal end opening 24. In addition, there is a gap between the inner circumferential surface of the syringe proximal end 21 and the outer circumferential surface of the plunger proximal end 31.

The plunger 3 can move upward with respect to the syringe 2 from the state shown in FIG. 2. When the plunger 3 moves upward by a predetermined length, the plunger distal end 34 will be submerged inside the tubular passage 2P. At this time, suction force is generated in the distal end opening 24, and the fluid around the distal end opening 24, the culture medium L including the cells C in the present embodiment can be suctioned into the tubular passage 2P. After this suction, when the plunger 3 moves downward, the fluid suctioned into the tubular passage 2P can be discharged from the distal end opening 24.

The suction tip 1 is attached to the distal end of the head 61 shown in FIG. 2. The head 61 includes a first cylindrical rod 611, a second cylindrical rod 612 placed outside of the first cylindrical rod 611, and a plunger rod 613 (generating mechanism/moving member) placed in the hollow part of the first cylindrical rod 611.

While the second cylindrical rod 612 is a fixed rod, the plunger rod 613 and the first cylindrical rod 611 each move back and forth independently. The plunger proximal end 31 includes a mounting hole 3H, a cylindrical hollow space. The lower end of the plunger rod 613 is press-fitted into the mounting hole 3H. The upper end surface of the plunger proximal end 31 faces the lower end surface of the first cylindrical rod 611. The lower end of the fixed second cylindrical rod 612 is press-fitted into the hollow part 2H of the syringe proximal end 21. By the up-and-down movement of the plunger rod 613, the plunger 3 moves up and down, generating suction force or discharge force in the distal end opening 24 as described above. The first cylindrical rod 611 is lowered when removing the suction tip 1 from the head 61.

### [Electric Configuration of Cell Transfer Apparatus]

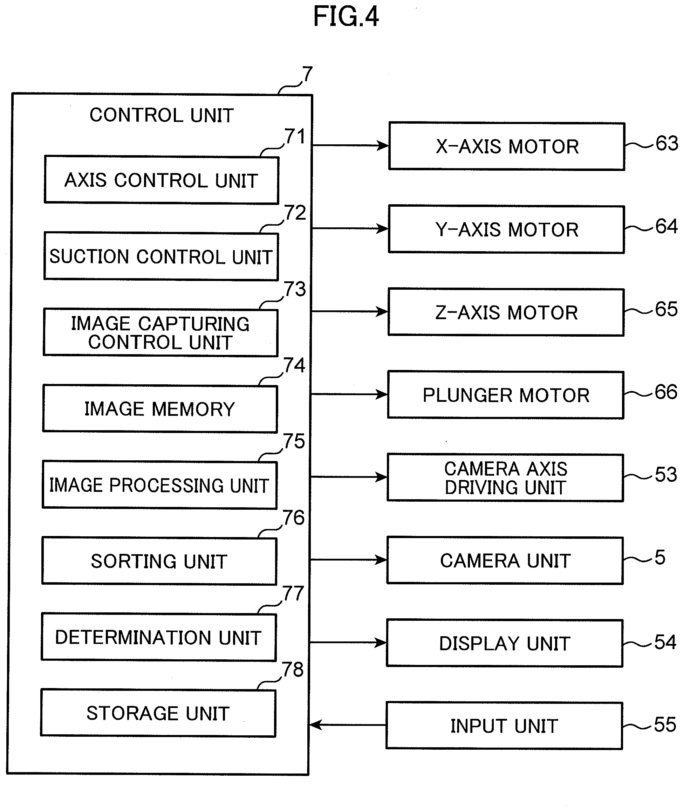

FIG. 4 is a block diagram showing the electric configuration of the cell transfer apparatus S. The cell transfer apparatus S includes a control unit 7 that controls the movement operation of the head unit 6, the suction and discharge operation of the cells C by the suction tip 1, the movement and image capturing operation of the camera unit 5, and the like. In addition, the cell transfer apparatus S includes a camera axis driving unit 53 as a mechanism for causing the camera unit 5 to horizontally move, an X-axis motor 63 and Y-axis motor 64 as mechanisms for horizontally moving the head unit 6, a Z-axis motor 65 as a mechanism for raising and lowering the head 61, and a plunger motor 66 as a mechanism for performing suction and discharge operations of the cells C, and further includes a display unit 54 and an input unit 55.

The camera axis driving unit 53 includes a driving motor for causing the camera unit 5 to horizontally move along the guide rail 5G. The X-axis motor 63 and the Y-axis motor 64 are driving sources for the movement mechanism that causes the head unit 6 to move horizontally along the guide rail 6G. The preferred mode of the movement mechanism includes ball screws and nut members for the X axis and Y axis, and the X-axis motor 63 and the Y-axis motor 64 each rotate the ball screw forward or backward.

The Z-axis motor 65 and the plunger motor 66 are built into the head body 62. The Z-axis motor 65 causes the head 61 to move up and down between the lowered position where the head 61 extends downward from the head body 62 and the raised position where most of the head 61 is accommodated in the head body 62. The plunger motor 66 generates suction force and discharge force in the distal end opening 24 of the suction tip 1 by raising and lowering the plunger rod 613 placed in the head 61 (FIG. 2).

The display unit 54 includes a liquid crystal display or the like, and displays images captured by the camera unit 5 and images that undergo image processing or the like by the control unit 7.

The input unit 55 includes a keyboard, touch panel, a communication unit for data communication with other communication devices, or the like, and receives input of operation information and various data from a user. In the present embodiment, the input unit 55 receives input of the set quantity of the cells C to be discharged into one well 45 of the destination second container 42 from the user. In other words, the input of the set quantity of the target cells C to be suctioned by one suction tip 1 in the first container 41 is received from the user.

The control unit 7 includes a processor or the like that performs various arithmetic processes, and includes an axis control unit 71 (part of the movement mechanism), a suction control unit 72, an image capturing control unit 73, an image memory 74, an image processing unit 75 (part of the cell recognition unit), a sorting unit 76, a determination unit 77, and a storage unit 78.

The axis control unit 71 controls the operation of the X-axis motor 63 and the Y-axis motor 64 to cause the head unit 6 to move to a predetermined horizontal target position. The movement of the head 61 to which the suction tip 1 is attached between the first container 41 and the second container 42, positioning perpendicularly above the accommodation sections 44 of the dish 43, positioning perpendicularly above the well 45 of the second container 42 where discharge is performed, and the like are implemented by controlling the X-axis motor 63 and the Y-axis motor 64 by the axis control unit 71.

The suction control unit 72 controls the operation of the Z-axis motor 65 and the plunger motor 66 to perform the suction and discharge operation of the cells C by the suction tip 1. The suction control unit 72 controls the operation of the Z-axis motor 65 to raise and lower the head 61 to be controlled toward a predetermined target position. The suction control unit 72 controls the operation of the plunger motor 66 equipped on the head 61 to be controlled to generate suction force or discharge force in the distal end opening 24 of the suction tip 1 at a predetermined timing.

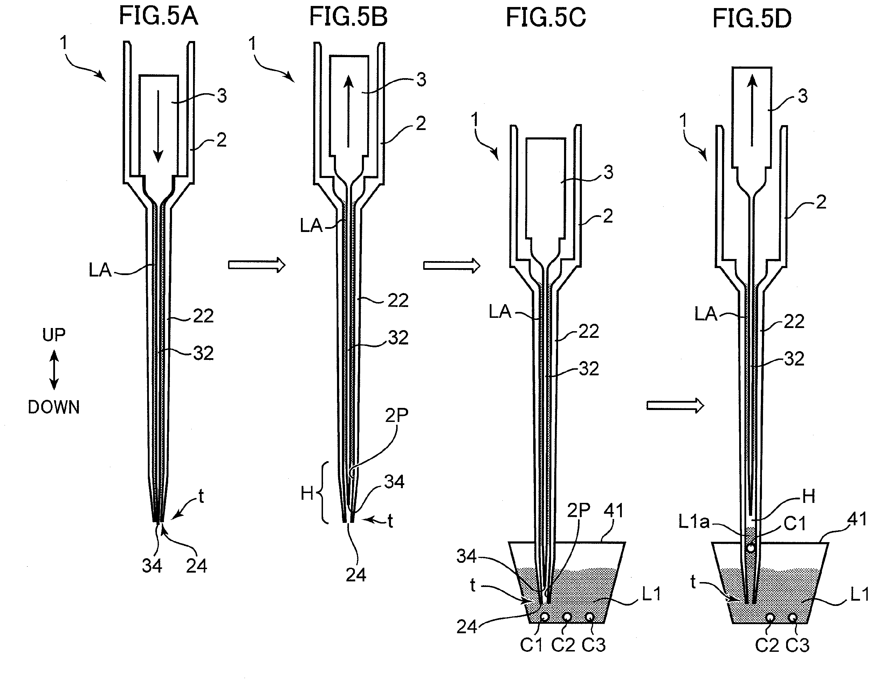

In the present embodiment, the suction control unit 72 performs an operation of gradually suctioning the set quantity of the target cells C that is set to two or more in advance from among the cells C scattered on the dish 43 of the first container 41 into one suction tip 1. The set quantity is a quantity received in the input unit 55 from the user. Furthermore, after the axis control unit 71 cause the head 61 to move to the second container 42, the suction control unit 72 performs an operation of discharging the set quantity of the target cells C suctioned by the suction tip 1 into the same location in the second container 42, that is, one well 45. Such control will be described using diagrams later with reference to FIGS. 5 to 7.

The image capturing control unit 73 controls the camera axis driving unit 53 to perform the operation of causing the camera unit 5 to move along the guide rail 5G. In addition, the image capturing control unit 73 controls the image capturing operation of the first container 41 or the second container 42 by the camera unit 5.

The image memory 74 is a storage area included in the microcomputer, an external storage, or the like. Image data acquired by the camera unit 5 is temporarily stored in the image memory 74.

The image processing unit 75 performs image processing on image data captured by the camera unit 5 and stored in the image memory 74. The image processing unit 75 performs processing to recognize the position on the image of the cells C on the dish 43 based on the image of the dish 43 of the first container 41 after the cells C are dispensed. In addition, the image processing unit 75 performs processing to recognize the distribution of the cells C, to recognize the size, shape, color tone, and the like of the recognized cells C, and the like by using the image processing technology. The axis control unit 71 and the suction control unit 72 control the movement of the head 61 and the suction and discharge of the suction tip 1 based on the position recognition of the cells C, and perform the transfer operation of the cells C.

The sorting unit 76 performs processing to sort the target cells C to be transferred to the second container 42 from a group of the cells C scattered in the first container 41 based on sorting criteria determined in advance. The sorting unit 76 derives the quality evaluation value of the cells C scattered on the dish 43 of the first container 41 based on the image captured by the camera unit 5. Specifically, based on image processing results by the image processing unit 75, for example, the feature amounts of the cell C such as the area or estimated volume of the cell C, the color or pattern of the cell C, and the light intensity when the cell C is fluorescent are extracted. In addition, the number of cells C accommodated in one accommodation section 44 is also detected as one of the feature amounts. This is because when multiple cells C are accommodated in the accommodation section 44, it is difficult to control the number of cells C suctioned to the suction tip 1, the cells C overlap, making accurate evaluation difficult, and other reasons.

Thereafter, the sorting unit 76 identifies the cell with the evaluation value exceeding a predetermined threshold as the target cell C. That is, the extracted feature amount of each cell C is compared with a selection criteria parameter that is determined in advance as the range of cells C to be selected, and the target cell C is identified by evaluating whether the cell falls within the range of the selection criteria parameter. Through the processing by the sorting unit 76, only the cell C that is evaluated as having superior quality among the cells C accommodated in the first container 41 can be transferred to the second container 42 as the target cell C.

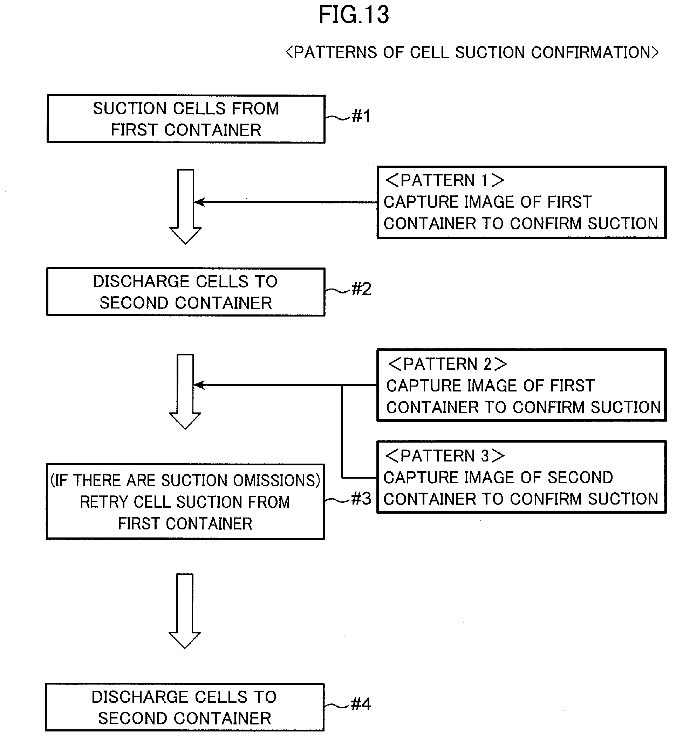

Based on the image of the first container 41 after the suction tip 1 performs the cell suction operation, or the image of the second container 42 after the suction tip 1 performs the cell suction operation, the determination unit 77 determines whether the suction or discharge of the set quantity of the target cells C is achieved. This determination pattern will be described in detail later with reference to FIG. 13.

The storage unit 78 stores various setting values and data in the cell transfer apparatus S. In addition, the storage unit 78 also stores the selection criteria parameter of the cell C to be transferred. The criteria for determining whether the cell C is good or bad may differ depending on the size and type, and, for example, it is preferable to have sorting criteria obtained from results of machine learning for each size and type. The sorting unit 76 reads the sorting criteria stored in the storage unit 78 during the sorting process.

### [Cell Transfer Operation]

Subsequently, the cell transfer operation by the cell transfer apparatus S will be described with reference to FIGS. 5 to 7. The cell transfer operation includes the following steps (1) to (4) to be performed sequentially.
(1) A preliminary treatment step of causing the suction tip 1 to hold a preliminary treatment liquid,
(2) A suction step of suctioning the set quantity of the target cells C into the suction tip 1 in the first container 41,
(3) A movement step of moving the suction tip 1 that has suctioned the cells C to the second container 42, and
(4) A discharge step of discharging all the cells C in the suction tip 1 into the well 45 of the second container 42.

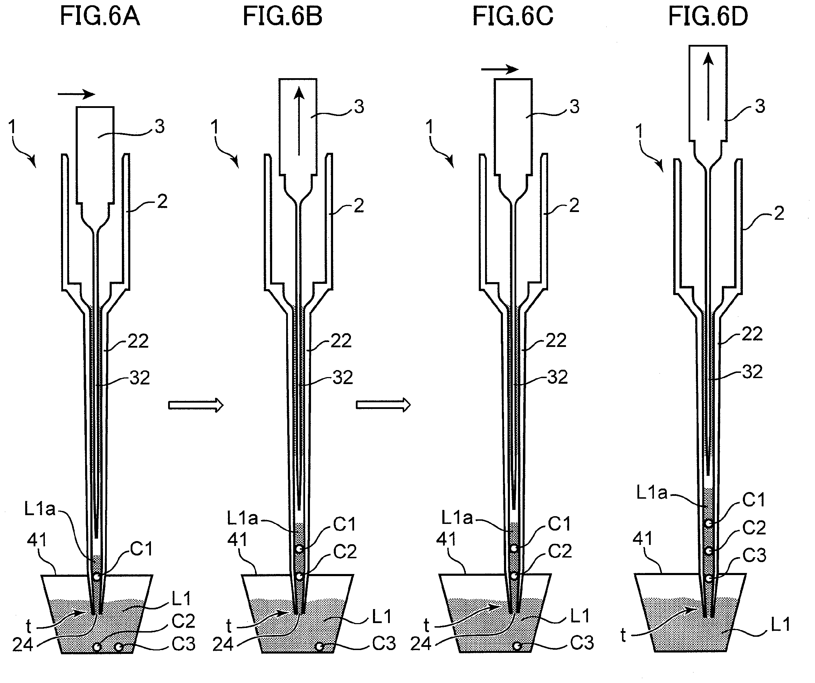

FIGS. 5A to 5D and FIGS. 6A to 6D are diagrams showing the above-mentioned preliminary treatment step (1) and suction step (2). FIG. 5A shows the suction tip 1 in a state of holding the preliminary treatment liquid LA in the space between the syringe body 22 and the plunger body 32. In the preliminary treatment step (1), for example, the distal end t of the syringe 2 is immersed in a container filled with the preliminary treatment liquid LA, and the plunger 3 moves back and forth about three times. This operation brings the preliminary treatment liquid LA into the space between the syringe body 22 and the plunger body 32. A physiological saline solution or the like may be used as the preliminary treatment liquid LA, but it is preferable to use the culture medium L.

FIGS. 5A and 5B are also diagrams showing the step of forming an air layer H inside the tubular passage 2P before performing the suction step (2). The suction control unit 72 places the distal end t of the suction tip 1 in the air as shown in FIG. 5A, and drives the plunger motor 66 from the state where the plunger distal end 34 protrudes from the distal end opening 24 of the syringe 2 to raise the plunger 3 by a predetermined length. As a result, the air layer H is formed in the tubular passage 2P near the distal end opening 24.

FIGS. 5C to 6D show the execution status of the suction step (2) sequentially. In these figures, the first container 41 is shown in a simplified manner. In the culture medium L1 stored in the first container 41, the first cell C1, the second cell C2, and the third cell C3 selected as target cells to be transferred are scattered. Here, an example is shown where the set quantity of the target cells = 3. Based on the image of the first container 41 captured by the camera unit 5 before cell suction, the position coordinates of each of the cells C1, C2, and C3 are determined.

The suction control unit 72 causes one suction tip 1 to suction the cells C1, C2, and C3 gradually. At this time, it is determined in what order to suction the cells C1, C2, and C3, that is, how to set the movement route for the head 61. As the movement route, it is preferable to select a route that is the shortest movement distance by referring to the position coordinates of the cells C1, C2, and C3 in order to shorten the tact time. Here, the three target cells lined up horizontally are gradually suctioned in the order of the first cell C1, the second cell C2, and the third cell C3.

FIG. 5C shows the state where the distal end opening 24 of the suction tip 1 is aligned with the first cell C1. The axis control unit 71 causes the head unit 6 to move such that the suction tip 1 is positioned above the first container 41. At this time, the head 61 moves such that the distal end opening 24 of the suction tip 1 is positioned vertically above the first cell C1 by referring to the position coordinates of the first cell C1. The suction control unit 72 drives the Z-axis motor 65 to lower the head 61 until the distal end t is immersed in the culture medium L1 in the first container 41, in more detail, until the distal end opening 24 is positioned near immediately above the first cell C1 in the culture medium L1. FIG. 5C shows the state after the head is lowered.

Subsequently, the suction control unit 72 drives the plunger motor 66 to raise the plunger 3 by a predetermined distance. As a result, the distal end opening 24 is depressurized to generate suction force, causing a part of the culture medium L1 and the first cell C1 in the first container 41 to be suctioned from the distal end opening 24 into the tubular passage 2P. In the tubular passage 2P, the first cell C1 will float in the suctioned culture medium L1a. FIG. 5D shows the state after the first cell C1 is suctioned.

In the suction step (2), since the preliminary treatment liquid LA is held in advance in the space between the syringe body 22 and the plunger body 32, the culture medium L1 will not rise rapidly in the tubular passage 2P due to capillary action or internal pressure. The air layer H functions as a sealing layer that separates the preliminary treatment liquid LA and the culture medium L1a containing the first cell C1 that is subsequently suctioned in the tubular passage 2P. Therefore, the suctioned first cell C1 does not transition into the preliminary treatment liquid LA. Therefore, it is possible to prevent the first cell C1 from being sandwiched between the syringe body 22 and the plunger body 32. Furthermore, the presence of the air layer H makes it possible to prevent the preliminary treatment liquid LA from mixing with the culture medium L1 or prevent the preliminary treatment liquid LA from being discharged into the second container 42 in the subsequent discharge step (4).

Hereinafter, similar operations are repeated as many times as the set quantity of the target cells. FIG. 6A shows the state where the head 61 moves horizontally a small distance and the distal end opening 24 of the suction tip 1 is aligned with the second cell C2. After that, the suction control unit 72 drives the Z-axis motor 65 to lower the head 61 until the distal end opening 24 of the suction tip 1 is positioned near immediately above the second cell C2. Subsequently, the suction control unit 72 drives the plunger motor 66 to raise the plunger 3 by a predetermined distance and generate suction force in the distal end opening 24 to suction the second cell C2 from the distal end opening 24. FIG. 6B shows the state after the second cell C2 is suctioned. In this stage, the first cell C1 and the second cell C2 are held in the suction tip 1 in a manner of being arranged up and down.

FIG. 6C shows the state where the head 61 moves further horizontally a small distance and the distal end opening 24 of the suction tip 1 is aligned with the third cell C3. The suction control unit 72 drives the Z-axis motor 65 to lower the head 61 until the distal end opening 24 of the suction tip 1 is positioned near immediately above the third cell C3. Subsequently, the suction control unit 72 drives the plunger motor 66 to raise the plunger 3 further by a predetermined distance and generate suction force in the distal end opening 24 to suction the third cell C3 from the distal end opening 24. FIG. 6D shows the state after the third cell C2 is suctioned. In this example, the gradual suction of the target cells is completed here. In the state where the suction step (2) is completed, the first cell C1, the second cell C2, and the third cell C3 are held to be aligned in an up-and-down row inside the suction tip 1.

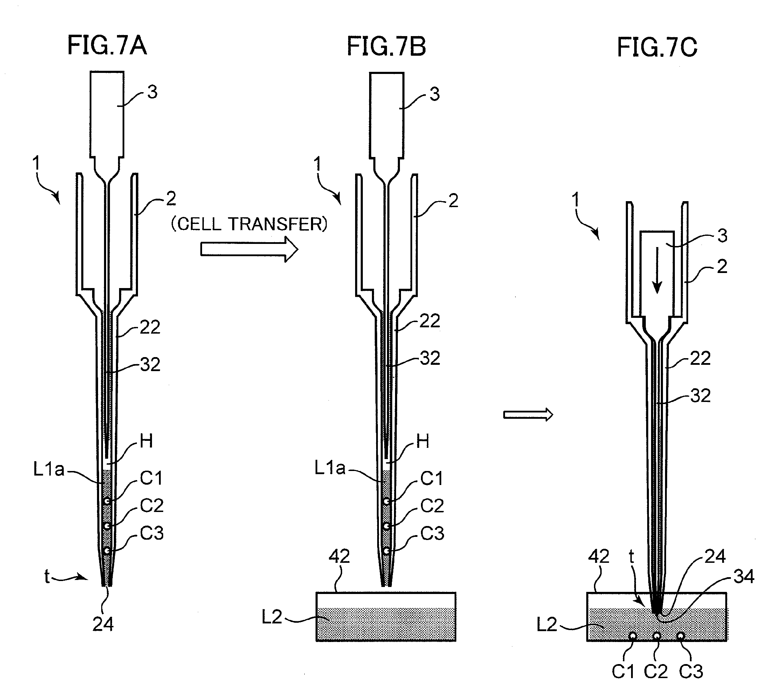

FIGS. 7A and 7B are diagrams showing the status of performing the movement step (3). After the completion of the suction step (2), the axis control unit 71 causes the head unit 6 to move such that the suction tip 1 holding the cells C1, C2, and C3 is positioned above the second container 42. In FIG. 7B, the second container 42 is schematically depicted. The culture medium L2 is stored in the second container 42 as well. Actually, the head unit 6 moves such that the distal end t of the suction tip 1 is aligned with one well 45 among the plurality of wells 45 included in the second container 42, the one well being the target for discharging.

FIG. 7C is a diagram showing the status after the discharge step (3) is performed. The suction control unit 72 drives the Z-axis motor 65 to lower the head 61 until the distal end opening 24 of the suction tip 1 is immersed in the culture medium L2 in the second container 42. Subsequently, the suction control unit 72 drives the plunger motor 66 to lower the plunger 3 by a predetermined distance. In more detail, the plunger 3 is lowered until the plunger distal end 34 protrudes from the distal end opening 24. As a result, as shown in FIG. 7C, the culture medium L1a and the cells C1, C2, and C3 held in the suction tip 1 are discharged all at once into the culture medium L2 in the second container 42.

FIGS. 8A and 8B are examples of images of the first container 41 having the accommodation sections 44 before and after the cell suction. The first container 41 includes the dish 43 having matrix-arrayed accommodation sections 44, and numerous cells C are scattered in the dish 43. As shown in FIG. 8A, it is assumed that, among these cells C, the cells Ca, Cb, and Cc accommodated one by one in the accommodation sections 44a, 44b, and 44c, respectively, are identified as target cells to be suctioned to one suction tip 1.

In this case, the suction control unit 72 sets a movement route MR that passes through the accommodation sections 44a, 44b, and 44c in the shortest distance as a movement route for the head 61 with the one suction tip 1 attached. Then, while the axis control unit 71 causes the head 61 to intermittently move along the movement route MR, the suction control unit 72 causes the one suction tip 1 to gradually suction the cells Ca, Cb, and Cc. That is, until the suction tip 1 finishes suctioning the set quantity of the target cells, the suction control unit 72 causes the head 61 to move across the plurality of accommodation sections 44a, 44b, 44c to cause the suction tip 1 to perform the suction operation.

After the suction, as shown in FIG. 8B, the accommodation sections 44a, 44b, and 44c become empty. If the cells Ca, Cb, and Cc remain in the accommodation sections 44a, 44b, and 44c, the suction will be considered a failure. That is, by capturing an image of the first container 41 after the cell suction and confirming the presence of the cells Ca, Cb, and Cc, it is possible to confirm whether the suction of the target cells by the suction tip 1 is successful or not.

The suction tip 1 that has suctioned the cells Ca, Cb, and Cc moves to the second container 42, discharging the cells Ca, Cb, and Cc into one well 45. FIG. 8C is an example of an image of the one well 45 after the cell discharge. In the well 45 that accommodates the cells Ca, Cb and Cc, necessary operations such as introducing reagents are performed.

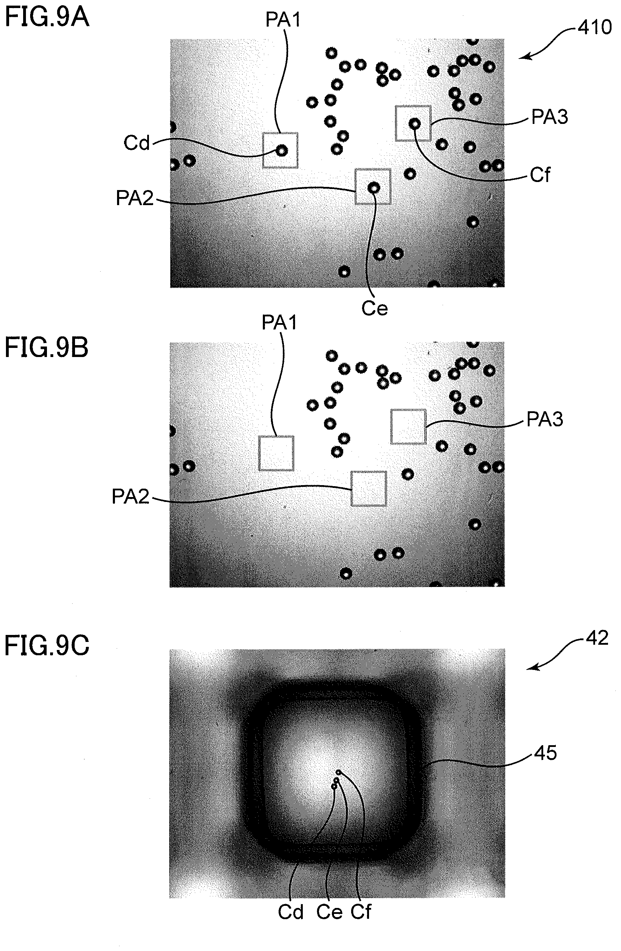

FIGS. 9A and 9B are examples of images of a first container 410 with a flat bottom having no accommodation sections 44 before and after cell suction. Numerous cells C are scattered in the first container 410. As shown in FIG. 9A, it is assumed that, among the cells C, cells Cd, Ce, and Cf that exist one by one in regions PA1, PA2, and PA3 respectively are identified as target cells to be suctioned into one suction tip 1. In this case as well, a movement route that passes through the regions PA1, PA2, and PA3 in the shortest distance is defined, and the cells Cd, Ce, and Cf are gradually suctioned into one suction tip 1. Whether the suction is successful can be determined, for example, by image matching processing of the pre-suction image in FIG. 9A and the post-suction image in FIG. 9B. FIG. 9C is an example of an image after the cells Cd, Ce, and Cf are discharged into one well 45.

The above is an operation focused on one suction tip 1. Actually, the set quantity of the target cells C in the first container 41 is gradually suctioned into each suction tip 1 attached to the multiple heads 61 included in the head unit 6. Then, the set quantity of the target cells C is discharged all at once from each suction tip 1 into each of the plurality of wells 45 of the second container 42. That is, two or more target cells C held in each suction tip 1 are discharged into the well 45 assigned to each suction tip 1. According to the present embodiment, it is possible to transfer the set quantity of the target cells C from each suction tip 1 to each well 45 of the second container 42 in one movement operation of the head unit 6 from the first container 41 to the second container 42.

### [Operation Flow of Cell Transfer Apparatus]

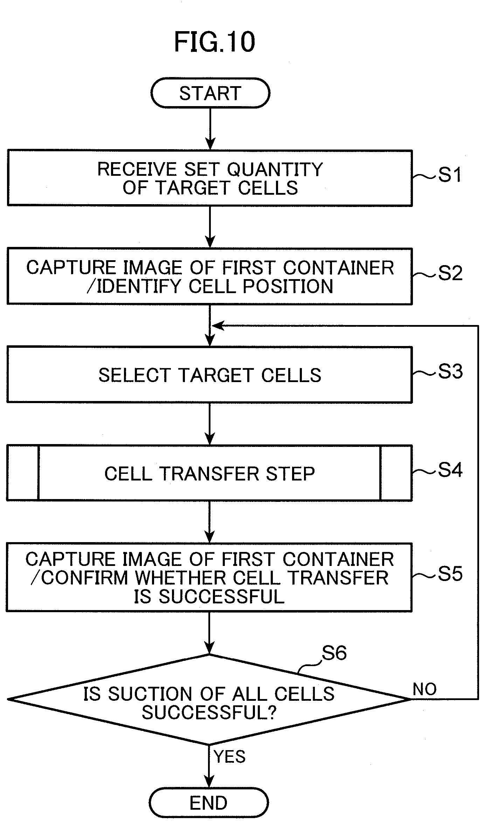

FIG. 10 is a flowchart showing an operation example of the cell transfer apparatus S. First, the input unit 55 receives the set quantity of the target cells C to be suctioned by one suction tip 1 from the user (step S1). The examples in FIG. 5 to FIG. 7 described above show the example where the set quantity = 3. It is preferable to make a selection from the range of about the set quantity = 2 to 5 because too large set quantity makes it difficult to suction the cells C into one suction tip 1.

Next, the image capturing control unit 73 controls the camera unit 5 to capture an image of the first container 41 before cell suction. The image processing unit 75 performs image processing on data of the acquired pre-suction image to identify the position of the cells C scattered in the first container 41 (step S2). Subsequently, the sorting unit 76 derives an evaluation value about the quality of the cells C based on the pre-suction image, and identifies the cells with the evaluation value exceeding a predetermined threshold as target cells C to be transferred (step S3).

Next, under the control of the axis control unit 71 and the suction control unit 72, the cell transfer step including the above-described preliminary treatment step (1), suction step (2), movement step (3), and discharge step (4) is performed (step S4). After the suctioned target cells C are discharged into the second container 42, the image capturing control unit 73 controls the camera unit 5 to capture images of the first container 41 after the cell suction. Then, by comparing the pre-suction image acquired in step S2 with the post-suction image acquired in this step, the determination unit 77 confirms whether the suction of the target cells C is successful, that is, whether the transfer of the target cells is successful (step S5).

When all the set quantity of the target cells C is successfully suctioned (YES in step S6), the process ends. On the other hand, when the suction of the target cells C fails partially or completely (NO in step S6), returning to step S3, the cell transfer step for recovery is performed again.

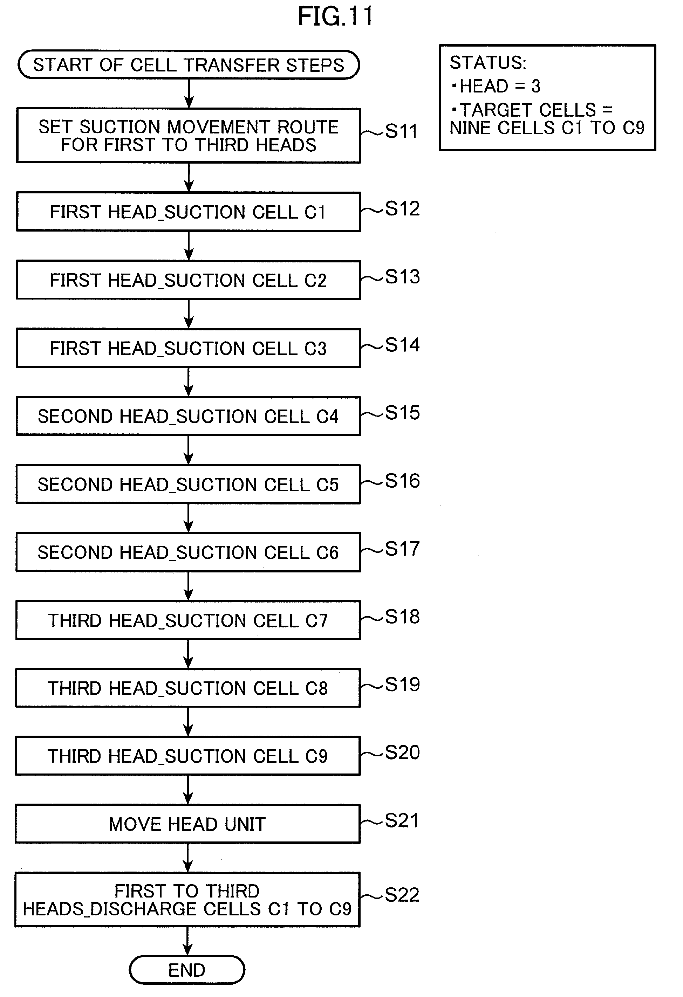

FIG. 11 is a flowchart showing the operation of the cell transfer step of step S4. Here, an example is shown where the head unit 6 includes three heads 61, there are nine cells to be transferred, cells C1 to C9, and each of the suction tips 1 attached to each head 61 suctions three cells (set quantity of target cells = 3). Note that the three heads 61 described above are referred to as first head, second head, and third head in the flowchart of FIG. 11.

When the target cells C1 to C9 are selected in step S3 of FIG. 10, the suction control unit 72 sets the movement route for the first head, second head, and third head on the first container 41 for suctioning these target cells (step S11). After that, while causing the first to third heads to move along the set movement route, the suction control unit 72 sequentially suctions the target cells C1 to C9, three at a time per one suction tip 1.

First, the suction control unit 72 causes the suction tip 1 attached to the first head to gradually suction the cells C1, C2, and C3 (steps S12, S13, S14). This series of suction operations involves the up-and-down and horizontal movement of the first head. Using the example of the dish 43 shown in FIG. 8A, the suction operation including the descent of the first head, suction of the cell Ca (C1) into the suction tip 1, and the ascent of the first head is performed in the accommodation section 44a to be approached first (step S12). Subsequently, the horizontal movement of the first head above the accommodation section 44b to be approached second is performed, and the suction operation for the cell Cb (C2) is performed similarly (step S13). The horizontal movement of the first head and the suction operation on the cell Cc (C3) are performed on the accommodation section 44c to be approached third as well (step S14).

Subsequently, the suction control unit 72 causes the suction tip 1 attached to the second head to gradually suction the cells C4, C5, and C6 (steps S15, S16, S17). Furthermore, the suction control unit 72 causes the suction tip 1 attached to the third head to gradually suction the cells C7, C8, and C9 (steps S18, S19, S20). The operation in these steps is the same as the suction operation for the first head (steps S12 to S14).

After completing the suction of the target cells C1 to C9 into the suction tip 1 of each of the first to third heads, the axis control unit 71 causes the head unit 6 to move above the second container 42 (step S21). After that, the suction control unit 72 discharges the target cells C1 to C9 from the suction tip 1 of each of the first to third heads into the designated well 45 (step S22). In more detail, the suction tip 1 of the first head is aligned with the first well 45 designated in advance, and the cells C1, C2, and C3 are discharged into the first well 45. Similarly, the cells C4, C5, and C6 are discharged from the suction tip 1 of the second head to the second well 45 designated in advance, and the cells C7, C8, and C9 are discharged from the suction tip 1 of the third head to the third well 45 designated in advance.

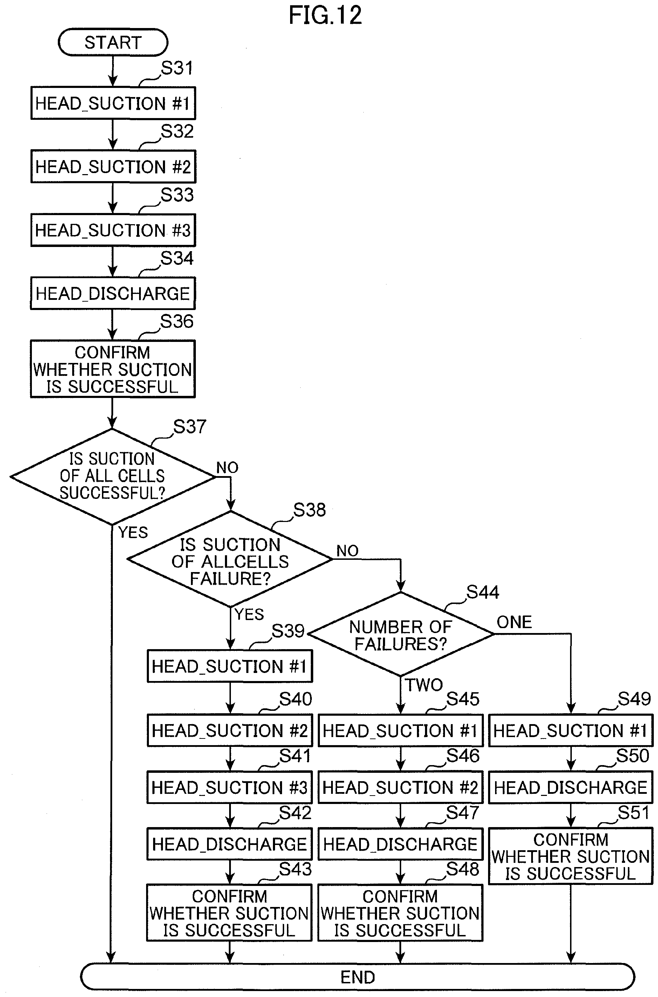

FIG. 12 is a flowchart showing details of the cell suction operation of the set quantity of the target cells C by the suction tip 1 of one head 61. The "head" in the flowchart of FIG. 12 corresponds to one of the first head, second head, or third head in the flowchart of FIG. 11. Here again, the set quantity of the target cells C to be suctioned by one suction tip 1 = 3.

The suction control unit 72 causes the head 61 to which the suction tip 1 is attached to perform three suction operations #1, #2, and #3 in the first container 41 according to the set quantity = 3 (steps S31, S32, S33). The suction operations #1, #2, and #3 here are the same as the suction operations described above in steps S12, S13, and S14. After that, the head 61 moves to the second container 42, and the suction control unit 72 discharges the target cells C suctioned in the suction operations #1, #2, and #3 into the well 45 determined in advance (step S34).

Subsequently, the determination unit 77 performs processing to determine whether the suction of the target cells C is successful based on the comparison of images of the first container 41 before cell suction and images of the first container 41 after cell suction, or images of the second container 42 after cell discharge (step S36). When all of the suction operations #1, #2, and #3 are successful (YES in step S37), the process for the head 61 this time (for example, first head) is completed, going to the process for the next head 61 (for example, second head). When all of the suction operations #1, #2, and #3 are not successful (NO in step S37), it is determined whether all of the suction operations #1, #2, and #3 have failed (step S38).

When all of the suction operations #1, #2, and #3 have failed (YES in step S38), the suction control unit 72 makes a retry of the suction operation. That is, the suction control unit 72 returns the head 61 to the first container, and causes the suction tip 1 attached to the head 61 to perform three suction operations #1, #2, and #3 on the target cells C (steps S39, S40, S41). Subsequently, the head 61 moves to the second container 42, and the suction control unit 72 discharges the target cells C suctioned in the retry suction operations #1, #2, and #3 into the well 45 determined in advance (step S42). After that, the determination unit 77 makes a determination whether the suction is successful (step S43). When all of the retry suction operations #1, #2, and #3 are successful, the process ends. After that, when all of the suction operations #1, #2, and #3 are not successful, the retry may be made again, or the well 45 may be treated as failure without making the retry again.

When all of the suction operations #1, #2, and #3 have not failed (NO in step S38), the number of failures of the suction operations is checked (step S44). When the suction of two target cells C has failed, the suction control unit 72 returns the head 61 to the first container, and causes the suction tip 1 attached to the head 61 to perform two retry suction operations #1 and #2 on the target cells C (steps S45, S46). Subsequently, the head 61 moves to the second container 42, and the suction control unit 72 discharges the target cells C suctioned in the retry suction operations #1 and #2 to the well 45 determined in advance (step S47). After that, the determination unit 77 makes a determination whether the suction is successful (step S48).

In step S44, when it is determined that the suction of one target cell C has failed, the suction control unit 72 returns the head 61 to the first container, and causes the suction tip 1 attached to the head 61 to perform one retry suction operation #1 on the target cell C (step S49). Subsequently, the head 61 moves to the second container 42, and the suction control unit 72 discharges the target cell C suctioned in the retry suction operation #1 to the well 45 determined in advance (step S50). After that, the determination unit 77 makes a determination whether the suction is successful (step S51).

### [Cell Suction Confirmation Pattern]

A variation of success or failure confirmation of cell suction by the determination unit 77 performed in step S5 of FIG. 10, step S36 of FIG. 12, or the like will be described with reference to FIG. 13. FIG. 13 illustrates three patterns, patterns 1 to 3. To summarize the flowchart in FIG. 12, the control unit 7 causes the head 61 to which the suction tip 1 is attached to perform the suction operation of the target cells C in the first container 41 (step #1), and causes the target cells C to be discharged into the second container 42 (step #2). If the suction fails, the head returns to the first container 41 and performs the retry suction operation on the target cells C (step #3), and causes the target cells C to be discharged again into the second container 42 (step #4).

The timing for the determination unit 77 to implement confirmation whether the cell suction is successful can be either between the steps #1 and #2 or after step #2. The former timing when capturing images of the first container 41 is "pattern 1", the latter timing when capturing images of the first container 41 is "pattern 2", and the latter timing when capturing images of the second container 42 is "pattern 3".

In both patterns 1 and 2, after the suction tip 1 suctions the set quantity of the target cells C from the first container 41, the camera unit 5 captures the post-suction image of the first container 41. The determination unit 77 compares the post-suction image and the pre-suction image of the first container 41 acquired before the suction operation to determine whether the cell suction is successful. In pattern 1, the confirmation whether success or failure is made before step #2. According to pattern 1, since success or failure becomes clear in the stage where the head 61 exists near the first container 41, it is possible to perform the retry suction as is when the suction failure is detected.

Meanwhile, in pattern 2, after step #2, that is, after the set quantity of the target cells C is discharged to the second container 42, the camera unit 5 captures the post-suction image of the first container 41, and the confirmation of success or failure is made. When the state where the suction tip 1 has suctioned multiple cells C continues for a long time, this can cause problems such as these cells C may settle and form clumps in the suction tip 1 or block the distal end opening 24. According to pattern 2, since the discharge of the suctioned target cells C into the second container 42 is prioritized, the occurrence of the above-described problems can be suppressed.

In pattern 3, after step #2, the camera unit 5 captures the post-discharge image of the second container 42 to confirm whether the suction is successful. That is, the determination unit 77 compares the post-discharge image and the pre-suction image to determine whether the cell suction is successful. In pattern 3, it is possible to confirm whether the discharge of the set quantity of the target cells C in the second container 42 is successful based on the post-discharge image. That is, the success or failure of the suction can be determined based on the result of discharge to the second container 42, enabling a more reliable determination.

### [Specific Example of Cell Transfer Apparatus]

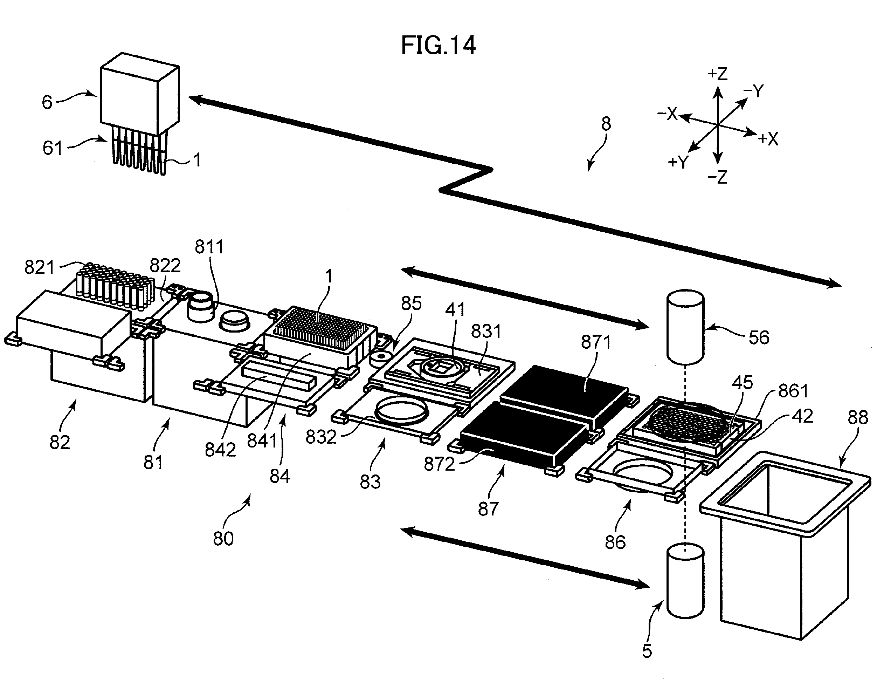

Subsequently, with reference to FIG. 14, a more specific cell transfer apparatus 8 into which the schematic cell transfer apparatus S shown in FIG. 1 is incorporated is illustrated. FIG. 14 is a perspective view showing a configuration example of the cell transfer apparatus 8. The cell transfer apparatus 8 includes a cell transfer line 80, the camera unit 5, the head unit 6, and a lighting unit 56. In FIG. 14, the base that supports the cell transfer line 80 and the movement mechanism of each unit are omitted.

The camera unit 5 and the head unit 6 are as described in FIG. 1. In FIG. 14, the head unit 6 including eight heads 61 to which the suction tips 1 are attached is illustrated. The head unit 6 is movable in the X and Y directions, and can move on the cell transfer line 80 along a predetermined movement path. In addition, the head 61 can rise and fall in the Z direction.

In the cell transfer line 80, elements necessary for implementation of a series of cell transfer steps of picking up cells accommodated in the source first container 41 and transferring the cells to the destination second container 42 are arranged in the X direction. In the cell transfer line 80, in order from the -X end, a dispenser tip stock unit 82, a cell stock unit 81, a tip stock unit 84, a tip image capturing unit 85, a cell sorting unit 83, a black cover placement unit 87, a cell relocation unit 86, and a tip disposal unit 88 are arranged in one line.

The cell stock unit 81 is a section that stores cell culture solution in which a large amount of cells are dispersed, which is the source of dispensing. The cell stock unit 81 includes a tube 811 that is a cylindrical container for storing the cell culture solution containing cells. The dispenser tip stock unit 82 is a section that stores a plurality of dispenser tips 821. The dispenser tip stock unit 82 is provided with a holder 822 that holds the dispenser tips 821 arranged in a matrix in an upright position.

The cell sorting unit 83 is a section for sorting cells of a desired size from the cell culture solution containing cells of various sizes. The cell sorting unit 83 includes the first container 41 that accommodates the cell culture solution, a holding table 831 that positions and holds the first container 41, and a table lid member 832 that covers an upper surface of the first container 41. The first container 41 preferably includes the dish 43, in a similar manner to the first container 41 shown in FIG. 1. An image of the cells carried in the first container 41 is captured by the camera unit 5 under lighting by the lighting unit 56. This identifies the location of the cells to be suctioned.

The tip stock unit 84 includes a holding box 841 that holds the large number of suction tips 1 described above arranged in a matrix. The suction tips 1 can be attached to and detached from the heads 61 of the head unit 6. The suction tips 1 perform the function of suctioning the cells carried in the first container 41, conveying the cells as the head unit 6 moves, and discharging the cells into the second container 42 in the cell relocation unit 86. The suction tips 1 are held in the holding box 841 in a state where attachment to the heads 61 that move in the Z direction can be easily made. In the tip stock unit 84, a reservoir 842 that stores impregnating liquid that moistens the distal end opening 24 of the suction tip 1 is also placed.

The tip image capturing unit 85 is a pit that provides a location where an image of the suction tip 1 attached to the head 61 is captured. The image capturing is performed by the camera unit 5. Based on the image of the suction tip 1 and the focal position information during image capturing, the XYZ coordinate position of the distal end opening 24 of the suction tip 1 is obtained. A correction value is derived from the difference between the coordinate position and a predetermined reference position, and is used as a correction value when controlling the movement of the head 61.

The cell relocation unit 86 is a section to which the cells suctioned by the suction tip 1 from the first container 41 of the cell sorting unit 83 are transferred. The cell relocation unit 86 includes the second container 42 and a holding table 861 that positions and holds the second container 42. The second container 42 is a plate with numerous wells 45 with an upper surface open as described above arranged in a matrix. The cells held by the suction tip 1 are discharged into the wells 45.

The black cover placement unit 87 is a section on which a first black cover 871 placed over the cell relocation unit 86 and a second black cover 872 placed over the cell sorting unit 83 are placed. The first and second black covers 871 and 872 are used when capturing images of cells carried in the first container 41 or the second container 42 in a state protected from light for cell fluorescence observation. The tip disposal unit 88 is a section where the suction tip 1 and the dispenser tip 821 are disposed of after use, after the cell suction and discharge operations are finished.

The cell transfer apparatus 8 includes a controller (not shown) that controls the overall operation of the cell transfer apparatus 8. The controller broadly causes the cell transfer apparatus 8 to perform the dispensing operation by the dispenser tip 821 and the cell transfer operation using the suction tip 1. The controller sequentially performs the following controls 1 to 4 in the dispensing operation.
[Control 1] Move the head unit 6 onto the dispenser tip stock unit 82 and attach the dispenser tip 821 to the dispensing nozzle (not shown) mounted on the head unit 6.
[Control 2] Move the head unit 6 onto the cell stock unit 81 to suction the cell culture solution containing the cells stored in the tube 811 into the dispenser tip 821 by a predetermined dispensing amount.
[Control 3] Move the head unit 6 onto the cell sorting unit 83 and discharge the cell culture solution in the dispenser tip 821 into the first container 41.
[Control 4] Move the head unit 6 onto the tip disposal unit 88, remove the used dispenser tip 821 from the dispensing nozzle, and dispose of the dispenser tip in the disposal unit 88.

The controller sequentially performs the following controls 5 to 8 in the cell transfer operation.
[Control 5] Move the head unit 6 onto the tip stock unit 84 and cause the suction tip 1 to be fitted onto the distal end of the head 61.
[Control 6] Move the head unit 6 onto the cell sorting unit 83 and suction the cells accommodated in the first container 41 into the suction tip 1. At this time, gradually suction the set quantity of the target cells that is set in advance to two or more into the suction tip 1.
[Control 7] Move the head unit 6 onto the cell relocation unit 86 and discharge the set quantity of the target cells in the suction tip 1 into the same location of the second container 42, that is, to one well 45.
[Control 8] Move the head unit 6 onto the tip disposal unit 88, remove the used suction tip 1 from the head 61, and dispose of the suction tip in the disposal unit 88.

With the cell transfer apparatus described above, one suction tip 1 suctions the set quantity of the target cells C together in the first container 41 to discharge the cells into one well 45 of the second container 41, thereby improving the efficiency of the transfer work of the cells C. In the conventional method, if multiple target cells C are required to be transferred to one well 45 of the second container 41, the head 61 has to reciprocate multiple times between the first container 41 and the second container 42, and the cell transfer work takes a lot of time. In contrast, with the cell transfer apparatus of the present embodiment, after the suction tip 1 sequentially suctions the set quantity of the target cells C in the first container 41, the head 61 moves to the second container 42 and then discharges the cells into one well 45 all at once. Therefore, the number of reciprocations of the head 61 between the first container 41 and the second container 42 can be reduced, and the time required for the cell transfer work can be shortened.

### [Invention Included in the Embodiment]

Note that the above-described specific embodiment mainly includes the invention having the following configurations.

A cell transfer apparatus according to one aspect of the present invention is a cell transfer apparatus for transferring cells scattered in a first container to a second container, and includes: a head to which a suction tip configured to suction and discharge the cells is attached, the head including a generating mechanism that generates suction force and discharge force in a distal end opening of the suction tip; a movement mechanism that causes the head to perform horizontal movement and up-and-down movement; a cell recognition unit that includes a camera that captures an image of the first container in which the cells are scattered, the cell recognition unit performing position recognition on the cells based on the image; and a control unit that controls the head and the movement mechanism to perform a transfer operation of the cells based on a result of the position recognition, in which the control unit causes the suction tip to gradually suction a set quantity of target cells from among the cells scattered in the first container, the set quantity being set in advance to two or more, and after causing the head to move to the second container, the control unit causes the suction tip to discharge the set quantity of the target cells that is suctioned by the suction tip into a same location in the second container.

With the cell transfer apparatus, the suction tip suctions the set quantity of the target cells together in the first container to discharge the cells into the same location of the second container, thereby improving the efficiency of the cell transfer work. In the conventional method, the suction tip suctions one target cell in the first container and moves to the second container, and discharges the one target cell into a specific location of the second container. In this method, if multiple target cells are required to be transferred to the specific location of the second container, the head has to reciprocate multiple times between the first container and the second container, and the cell transfer work takes a lot of time. In contrast, with the cell transfer apparatus described above, after the suction tip sequentially suctions the set quantity of the target cells in the first container, the head moves to the second container and then discharges the cells into the same location all at once. Therefore, the number of reciprocations of the head between the first container and the second container can be reduced, and the time required for the cell transfer work can be shortened.

In the cell transfer apparatus, the suction tip may include a syringe having a tubular passage connected to the distal end opening, and a plunger slidably accommodated in the tubular passage to generate negative pressure in the distal end opening, the head may include a moving member that causes the plunger to move forward and backward as the generating mechanism, and the control unit may perform control to repeat, for the set quantity, an operation to cause the head to move to align the distal end opening of the suction tip with the target cells and cause the plunger to move to generate the suction force in the distal end opening to suction the target cells into the suction tip.

According to this aspect, by simply controlling the forward and backward operation of the plunger, the sequential suction of the set quantity of the target cells into the suction tip and the discharge thereof can be performed.

The cell transfer apparatus may further include a head unit including a plurality of the heads, the second plate may include a plurality of accommodation portions into which the target cells are discharged, and the control unit may cause each suction tip of the plurality of heads to suction the set quantity of the target cells, and discharge the set quantity of the target cells from the each suction tip into each of the plurality of accommodation portions.

According to this aspect, it is possible to transfer the set quantity of the target cells from each suction tip to the plurality of accommodation portions in the second container in one movement operation of the head unit from the first container to the second container. Therefore, the time required for the cell transfer work can be further reduced.

In the cell transfer apparatus, the control unit may be configured to derive an evaluation value of quality of the cells scattered in the first container based on the image captured by the camera, and to identify the cells with the evaluation value exceeding a predetermined threshold as the target cells.

According to this aspect, only the cells that are evaluated as having superior quality among the cells accommodated in the first container can be transferred to the second container as the target cells.

In the cell transfer apparatus, the control unit preferably determines a movement route for the head when the set quantity of the target cells is gradually suctioned into the suction tip based on the image captured by the camera.

According to this aspect, it is possible to set an efficient movement route for the head by referring to the image of the first container. Therefore, the efficiency of the gradual suction operation of the target cells can be improved more than if the set quantity of the target cells is randomly suctioned into the suction tip.

In the cell transfer apparatus, the control unit may cause the camera to capture a post-suction image of the first container after performing an operation of suctioning the set quantity of the target cells from the first container to the suction tip, and determine whether the suction tip suctions the set quantity of the target cells based on the post-suction image.

According to this aspect, it is possible to confirm whether the suction of the set quantity of the target cells in the first container is successful based on the post-suction image. If it is determined that the suction has failed, it is possible to additionally transfer the missing target cells to a predetermined location in the second container.

In the cell transfer apparatus, after discharging the set quantity of the target cells into the second container, the control unit preferably causes the camera to capture the post-suction image of the first container.

When the state where the suction tip has suctioned multiple cells continues for a long time, this can cause problems such as these cells may settle and form clumps in the tip or block the distal end opening. According to the above aspect, since the discharge of the suctioned target cells into the second container is prioritized, the occurrence of the problems can be suppressed.

In the cell transfer apparatus, the control unit may cause the camera to capture a post-discharge image of the second container after performing an operation of discharging the set quantity of the target cells from the suction tip to the second container, and determine whether the suction tip discharges the set quantity of the target cells based on the post-discharge image.

According to this aspect, it is possible to confirm whether the discharge of the set quantity of the target cells in the second container is successful based on the post-discharge image. If it is determined that that discharge has failed, it is possible to additionally transfer the missing target cells to a predetermined location in the second container.

In the cell transfer apparatus, the first container may have a plurality of accommodation sections each capable of accommodating cells, and the control unit may cause the head to move across the plurality of accommodation sections and cause the suction tip to perform a suction operation until the suction tip finishes suctioning the set quantity of the target cells.

According to this aspect, it is possible to perform an operation of causing one suction tip to suction the target cells that exist in multiple accommodation sections and then discharging the target cells into the same location in the second container.

## Claims

1. cell transfer apparatus for transferring cells scattered in a first container to a second container, the cell transfer apparatus comprising:
a head to which a suction tip configured to suction and discharge the cells is attached, the head including a generating mechanism that generates suction force and discharge force in a distal end opening of the suction tip;
a movement mechanism that causes the head to perform horizontal movement and up-and-down movement;
a cell recognition unit that includes a camera that captures an image of the first container in which the cells are scattered, the cell recognition unit performing position recognition on the cells based on the image; and
a control unit that controls the head and the movement mechanism to perform a transfer operation of the cells based on a result of the position recognition,
wherein the control unit causes the suction tip to gradually suction a set quantity of target cells from among the cells scattered in the first container, the set quantity being set in advance to two or more, and
after causing the head to move to the second container, the control unit causes the suction tip to discharge the set quantity of the target cells that is suctioned by the suction tip into a same location in the second container.

2. The cell transfer apparatus according to claim 1, wherein
the suction tip includes a syringe having a tubular passage connected to the distal end opening, and a plunger slidably accommodated in the tubular passage to generate negative pressure in the distal end opening,
the head includes a moving member that causes the plunger to move forward and backward as the generating mechanism, and
the control unit repeats, for the set quantity, an operation to cause the head to move to align the distal end opening of the suction tip with the target cells and cause the plunger to move to generate the suction force in the distal end opening to suction the target cells into the suction tip.

3. The cell transfer apparatus according to claim 1 or 2, further comprising a head unit including a plurality of the heads,
wherein the second container includes a plurality of accommodation portions into which the target cells are discharged,
the control unit
causes each suction tip of the plurality of heads to suction the set quantity of the target cells, and
discharges the set quantity of the target cells from the each suction tip into each of the plurality of accommodation portions.

4. The cell transfer apparatus according to any one of claims 1 to 3, wherein the control unit derives an evaluation value of quality of the cells scattered in the first container based on the image captured by the camera, and identifies the cells with the evaluation value exceeding a predetermined threshold as the target cells.

5. The cell transfer apparatus according to any one of claims 1 to 4, wherein the control unit determines a movement route for the head when the set quantity of the target cells is gradually suctioned into the suction tip based on the image captured by the camera.

6. The cell transfer apparatus according to any one of claims 1 to 5, wherein
the control unit
causes the camera to capture a post-suction image of the first container after performing an operation of suctioning the set quantity of the target cells from the first container to the suction tip; and
determines whether the suction tip suctions the set quantity of the target cells based on the post-suction image.

7. The cell transfer apparatus according to claim 6, wherein after discharging the set quantity of the target cells into the second container, the control unit causes the camera to capture the post-suction image of the first container.

8. The cell transfer apparatus according to any one of claims 1 to 5, wherein
the control unit
causes the camera to capture a post-discharge image of the second container after performing an operation of discharging the set quantity of the target cells from the suction tip to the second container; and
determines whether the suction tip discharges the set quantity of the target cells based on the post-discharge image.

9. The cell transfer apparatus according to any one of claims 1 to 8, wherein
the first container has a plurality of accommodation sections each capable of accommodating cells, and
the control unit causes the head to move across the plurality of accommodation sections and causes the suction tip to perform a suction operation until the suction tip finishes suctioning the set quantity of the target cells.
